# EUROPEAN PATENT APPLICATION

(11) **EP 4 512 458 A1**
(43) Date of publication of application: **26.02.2025**
(21) Application number: 24187985.7
(22) Date of filing: 11.07.2024
(51) Int. Cl.: A61M 39/26

(54) **MEDICAL FLUID CONNECTOR**

(30) Priority: 23.08.2023 MY PI2023005058
(71) Applicant: Welford Manufacturing (M) Sdn Bhd, Sungai Buloh Selangor 47000 (MY)
(72) Inventor: WONG, Thean Fook, 60000 WILAYAH PERSEKUTUAN KUALA LUMPUR (MY); WONG, Lun Ting, 60000 WILAYAH PERSEKUTUAN KUALA LUMPUR (MY)
(74) Representative: Lavoix

(57) **Abstract**

The present invention relates to a fluid connector for medical use, more particularly to a the fluid connector configured with a multi-stage fluid control valve which is needle-less and provides a neutral pressure experience to a user. The fluid connector (100) comprises: two coaxial, interconnected end pieces (201, 202) which comprise the opposite ends of the connector, and through each of which extends a fluid passageway within each end piece (201, 202), said passageways together providing fluid communication between said opposite ends; a multi-stage fluid control valve (300) provided within the interconnected end pieces, the fluid control valve, when inactive, closes fluid flow through the fluid passageways of both end pieces (201, 202) and hence through the connector (100), said control valve (300) comprising a resiliently compressible assembly (301, 302) which can be activated such that a first predetermined compression of the resilient assembly (301, 302) puts the control valve (300) into a partial flow condition in which fluid flow is established into the fluid passageway of one a first of the end pieces (201), but holds fluid flow from egress through the fluid passageway of the opposite second end piece (202), and such that a continued further second predetermined compression of the resilient assembly (301, 302) puts the control valve (300) into a fully open condition allowing fluid flow through the fluid passageways of both the first (201) and second end pieces (202) and hence through the connector (100), and wherein disconnection of the connector (100) from said fluid pathway is affected by causing a multi-stage relaxation of the resilient assembly (301, 302) to a fully relaxed condition to close fluid flow through the connector (100).

## Description

### FIELD OF TECHNOLOGY

The present invention relates generally to medical devices, more specifically to fluid connector suitable for use in the medical field.

### BACKGROUND OF THE INVENTION

Needleless connectors for connecting intravenous (IV) catheters, administration sets, and syringes were introduced for the purpose of reducing the risks of needleprick injuries among health care providers. They are the devices that allow quick access to infusion line, without the need to use a needle. Generally, there are two major categories of needleless connectors, namely (1) simple connectors which have no internal moving parts, such as those with an external split septum; and (2) complex connectors, which rely on internal moving components, such as a mechanical valve, to control the flow of fluid within the device. Internal structures of the connectors determine the way the devices manage fluid displacement, as well as fluid pathway. American Journal of Nursing (AJN), Volume 112, No. 11 is referred herein for the relationship between fluid displacement and fluid pathway, as well as, meaning thereof, in relation to needleless connectors.

An exemplary needleless fluid connector is disclosed by Patent Cooperation Treaty Patent Publication No. 9850106 A1. Particularly, the connector comprises a movable diaphragm assembly and an internal central cannula. More particularly, the diaphragm is and includes cams formed therein, wherein the cams engage with a proximal portion of the internal cannula facilitates movement of the diaphragm past the proximal portion of the internal cannula. Another example is disclosed by United States Patent No. 9855412 B2, particularly a needleless valve for interconnecting with a blunt fluid supply nozzle, in which the valve comprises a base with a connector; a solid elongated fluid channeling rod; and a hollow flexible plug which provides a variable volume fluid flow path between its inlet and outlet ends, whereby the variable fluid flow path enables residual fluid therein to be expelled from the valve when the plug returns to its deactivated configuration.

### SUMMARY OF THE INVENTION

One aspect of the present invention is to provide a needleless connector suitable for use in association with an intravenous (IV) catheters, administration sets, and syringes, but not limited thereto. The connector may facilitate in administration of fluid substance into a patient, or retrieval of bodily fluid from a patient. For example, one side of the connector is configured to connect with a medical device or tool, such as but not limited to, a needleless syringe, an intravenous (IV) drip line, blood transfusion line, while another side of the connector is configured to connect with an adapter on a patient, such as a catheter, luer lock extension set.

One aspect of the present invention is to provide a two-way fluid connector configured with a valve which may be repeatedly opened and closed. More particularly, the connector is configured with a housing; and a multi-stage fluid control valve comprising a resiliently compressible assembly, in which the housing and the resiliently compressible assembly can be work in association with one another to provide a open or close position of the connector by providing at least a predetermined compression.

Another aspect of the present invention is to provide a neutral pressure fluid connector. More particularly, the connector is configured to provide neutral fluid displacement whereby it prevents blood from moving into catheter lumen upon connection or disconnection. To achieve that, the connector is configured with a multi-stage fluid control valve such that changes in pressure within the connector can be managed. Advantageously, inflow or egress of fluid may be experienced at a minimum level by the patient.

Another aspect of the present invention is to provide a swabable medical connectors. More particularly, the connectors can be swabbed in a conventional manner with a suitable disinfectant before use.

Another aspect of the present invention is to provide a backflow resistance fluid connector. More particularly, the connector is configured with a multi-stage fluid control valve such that backflow, such as those cause by syringe rebound or disconnection, can be captured there within.

Another aspect of the present invention is to provide a fluid connector configured with transparent material whereby fluid being transferred therein can be visible to the naked eye. More particularly, the transparent material used can be polycarbonate.

At least one of the preceding aspects is met, in whole or in part, by the present invention, in which one of the embodiments of the present invention is a fluid connector (100) particularly for medical use as an interconnector in a fluid pathway, the fluid connector, when assembled, including; two coaxial, interconnected end pieces (201, 202) which comprise the opposite ends of the connector, and through each of which extends a fluid passageway within each end piece, said passageways together providing fluid communication between said opposite ends, a multi-stage fluid control valve (300) provided within the interconnected end pieces (201, 202), the fluid control valve, when inactive, closes fluid flow through the fluid passageways of both end pieces (201, 202) and hence through the connector (100), said control valve (300) comprising a resiliently compressible assembly (301, 302) which can be activated such that a first predetermined compression of the resilient assembly puts the control valve into a partial flow condition in which fluid flow is established into the fluid passageway of one a first of the end pieces (201), but holds fluid flow from egress through the fluid passageway of the opposite second end piece (202), and such that a continued further second predetermined compression of the resilient assembly puts the control valve into a fully open condition allowing fluid flow through the fluid passageways of both the first (201) and second end pieces (202) and hence through the connector (100), and wherein disconnection of the connector (100) from said fluid pathway is affected by causing a multi-stage relaxation of the resilient assembly (301, 302) to a fully relaxed condition to close fluid flow through the connector.

Preferably, the first end piece (201) is received at least partially within the second end piece (202), and wherein the first end piece (201) accommodates the resiliently compressible assembly (301, 302).

Preferably, the resiliently compressible assembly includes a first component (301) which controls fluid flow primarily within the fluid passageway of the first end piece (201) and a second component (302) which primarily controls fluid flow through the second end piece (202).

Preferably, the first component (301) is resiliently movable within the first end piece (201) to open and close fluid flow therethrough and wherein the second component (302) is resiliently movable also within the first end piece (201) to open and close fluid flow through the second end piece (202).

Preferably, a portion of the fluid passageway of the second end piece (202) extends within, and thereby provides a portion of, the fluid passageway of the first end piece (201).

Preferably, the second component (302) opens and closes fluid communication through the passageway portion extending within the first end piece which thereby controls fluid flow through both first (201) and second end pieces (202).

Preferably, a coaxial conduit element (400) is housed within the first end piece (201) and disposed between the first (301) and second components (302) of the resilient assembly, and wherein fluid flow through one end of the conduit element (400) is controlled by the first component (301) and fluid flow through the opposite end of the conduit element (400) is controlled by the second component (302).

Preferably, the arrangement is such that the first predetermined pressure causes compression of the first component (301) to open fluid communication of the first end piece (201) through the conduit element (400) and wherein the second predetermined pressure causes axial movement of the conduit element (400) and compression of the second component (302) to establish fluid flow through both end pieces (201, 202) and hence the connector (100).

Preferably, the second predetermined pressure causes fluid communication between the opposite end of the conduit element (400) and fluid passageway of the second end piece (302) of the connector.

### BRIEF DESCRIPTION OF THE DRAWINGS

The invention is illustrated, although not limited, by the following description of embodiments made with reference to the accompanying drawings in which:
Figure 1 is a perspective illustration depicting an embodiment of the assembled connector (100) in the present disclosure.
Figure 2 is a perspective cross-sectional illustration of Figure 1.
Figure 3 shows a side view of the embodiment of the assembled connector (100) in the present disclosure in a fully relaxed (close) position.
Figure 4 shows a side view of the embodiment of the assembled connector (100) in the present disclosure in a partially open position.
Figure 5 shows a side view of the embodiment of the assembled connector (100) in the present disclosure in a fully open position
Figure 6 is a perspective illustration depicting an exemplary first end piece (201) of the connector (100) in the present disclosure.
Figure 7 is a perspective cross-sectional illustration of Figure 6.
Figure 8 shows a side view of an exemplary first end piece (201) of the connector (100) in the present disclosure.
Figure 9 is a perspective illustration depicting an exemplary first component (301) of the resiliently compressible assembly in the present disclosure, in which the dotted lines depicts internal structure thereof.
Figure 10 shows a perspective cross-sectional illustration of Figure 9.
Figure 11 shows a side view of an exemplary first component (301) of the resiliently compressible assembly in the present disclosure.
Figure 12 is a perspective illustration depicting an exemplary coaxial conduit element (400) of the connector (100) in the present disclosure.
Figure 13 shows a perspective cross-sectional illustration of Figure 12.
Figure 14 shows a side view of an exemplary coaxial conduit element (400) of the connector (100) in the present disclosure.
Figure 15 shows a side view of a coaxial conduit element (400) fitted in the first component (301) of the resiliently compressible assembly in the present disclosure.
Figure 16 is a perspective illustration depicting an exemplary second component (302) of the resiliently compressible assembly in the present disclosure.
Figure 17 shows a perspective cross-sectional illustration of Figure 16.
Figure 18 a side view of an exemplary second component (302) of the resiliently compressible assembly in the present disclosure.
Figure 19 is a perspective illustration depicting an exemplary second end piece (202) of the connector in the present disclosure.
Figure 20 is a perspective cross-sectional illustration of Figure 19.
Figure 21 shows a side view of an exemplary second end piece (202) of the connector (100) in the present disclosure.

### DETAILED DESCRIPTION OF THE INVENTION

Exemplary, non-limiting embodiments of the invention will be disclosed. However, it is to be understood that limiting the description to the preferred embodiments of the invention is merely to facilitate discussion of the present invention and it is envisioned that those skilled in the art may devise various modifications without departing from the scope of the appended claim.

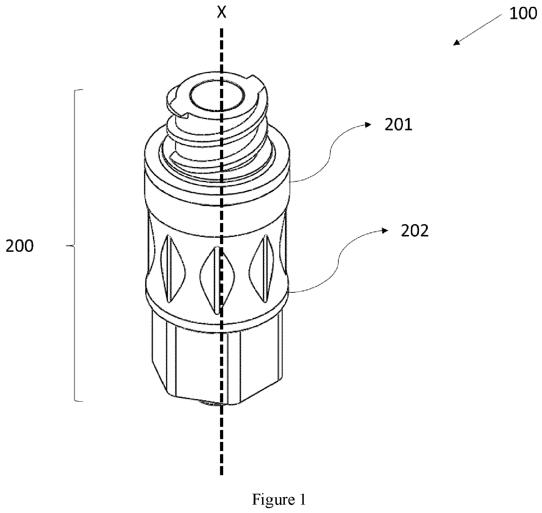

According to Figure 1, the connector (100) comprises a housing (200) having two opposite ends. One end of the connector is referred herein as proximal end while another end thereof is referred herein as distal end. When in use, the proximal end can be connected to suitable external medical devices or systems, such as but not limited to, blood transfusion sets, administration sets, syringes, while the distal end of the connector can be connected to an adapter on a patient, such as but not limited to, a catheter, luer lock extension set. In one embodiment, the proximal end of the housing (200) is configured from a first end piece (201) while the distal end of the housing (200) is configured from a second end piece (202). The first end piece (201) and the second end piece (202) have surface structures which are configured to be complementary with one another for a snug fit, or interlocking connection. Particularly, the first end piece (201) is received at least partially within the second end piece (202). In one embodiment, the first end piece (201) is partially fitted into the second end piece (202). Further, the first end piece (201) and the second end piece (202) are configured to selectively establish fluid passageway providing fluid communication between the proximal end and the distal end. In one embodiment, the first end piece (201) and the second end piece (202) are co-axial to one another, in which each end piece has individual inlet and outlet which are configured to be aligned along a common, central axis (X).

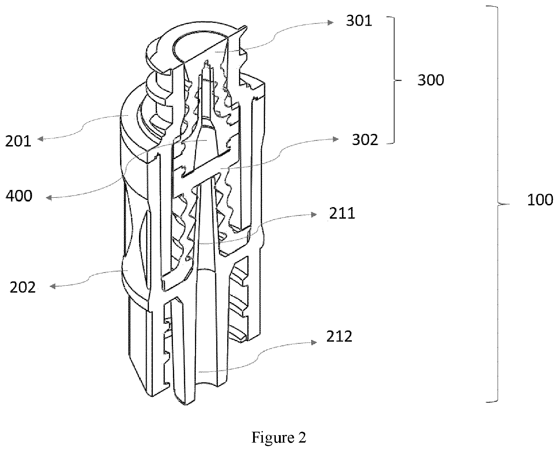

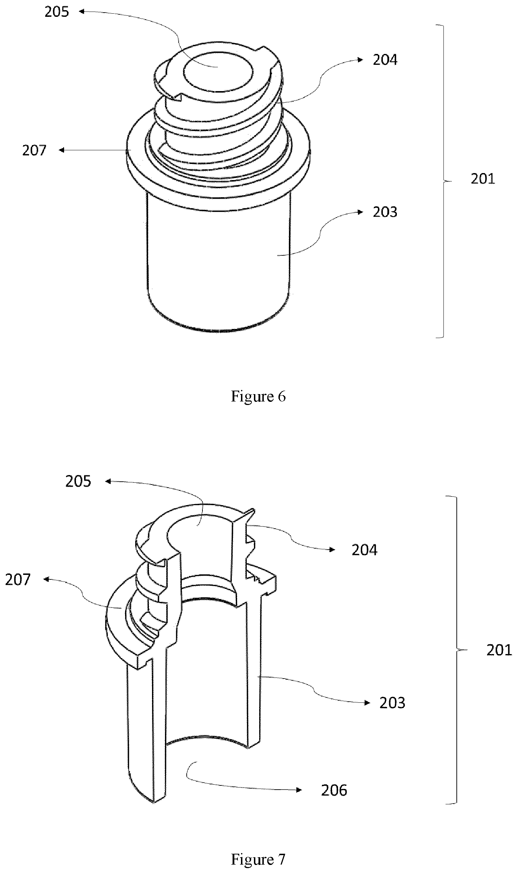

According to Figure 2, 6 to 8, the first end piece (201) has a first tubular body (203) with an extended neck portion (204). More particularly, external surface of the portion (204) is configured with screwthreaded pattern such as those in luer lock or slip for connection with external devices or systems. One end of the body proximal to the neck has an inlet opening (205) and the other, opposite end has an outlet opening (206). The inlet opening (205) serves as the proximal end of the connector, where it is configured to house a multi-stage valve (300) and provide access of the valve to an external device. The outlet opening (206) is substantially larger in diameter than the inlet opening (205), whereby it allows other components of the connectors to enter therefrom and be arranged therein. The tubular body (203) has a substantially smooth exterior surface while the extended neck portion (204) has an external flange (207) and a patterned external surface, such as screwthreaded, having raised ribs, or the like.

According to Figure 2, 19 to 21, the second end piece (202) has a second tubular body (208) corresponding to the first tubular body (203) in shape and slightly larger in diameter to allow fitting of the first tubular body (203) therein. The second tubular body (208) has an open end (209) and a base (210) opposite thereto, the base (210) is configured with an internal cannula (211). In one embodiment, the internal cannula (211) extends through the base and being configured with a luer end (212). The tubular body (208) of the second end piece (202) corresponds to the size and shape of the tubular body (203) of the first end piece (201) insofar that the first (201) and second end piece (202) can connect with one another by slip fitting or interlocking features. In one embodiment, the first end piece (201) slides into the second end piece (202) and stops when it reaches to the flange (207). In one embodiment, seal can be provided between the first end piece (201) and the second end piece (202) to enhance the connection. In accordance to the preceding description, the base (210) would act as the base for the housing (200) upon assembly. Further, the luer end (212) refers to the distal end of the connector (100) where it may connect with adapter on a patient. The second tubular body (208), the base (210) configured with internal cannula (211) and the luer end (212) are preferably integrated into a single piece. Preferably they are made from a relatively rigid material as compared to the compressible valve (300). In other embodiment, the second end piece (202) can be assembled from the aforementioned parts, whereby the internal cannula (211) and the luer end (212) are made from a relatively rigid material as compared to the compressible valve (300). Examples of the relatively rigid material are polycarbonate or acrylonitrile butadiene styrene (ABS).

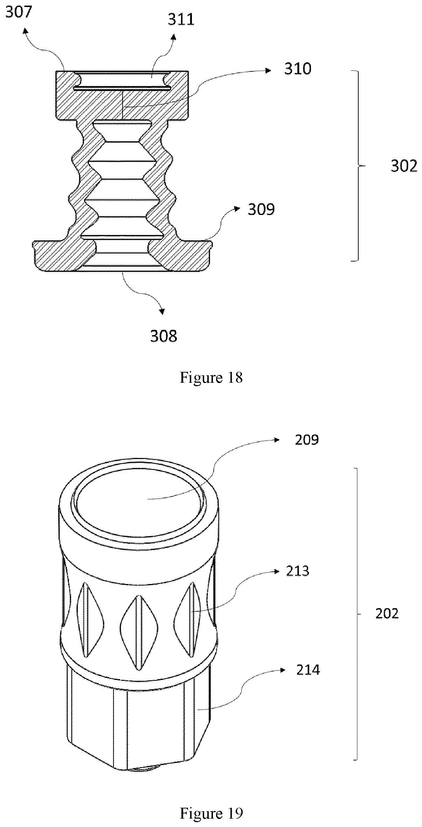

According to Figure 1 and 19, the second end piece (202) has an external surface configured with gripping feature (213). The gripping features can be protrusions or depressions arranged circumferentially about the external surface of the tubular body (208). It shall be understood that the pattern and arrangement of the gripping feature (213) may vary insofar to provide a good grip when it is in used.

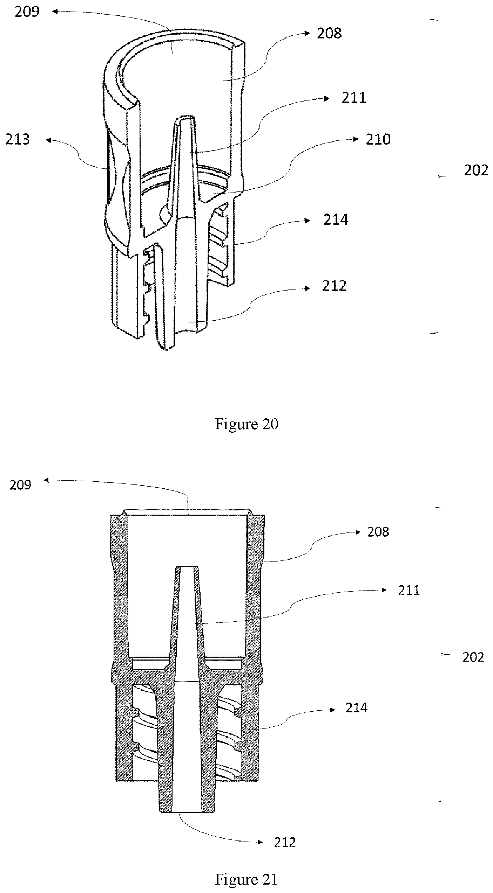

In one embodiment, the internal cannula (211) extends through the center of the base (210), in which the cannula (211) provides an internal channel for fluid flow there through, when activated. According to Figure 20 and 21, one side of the cannula (211) has an opening facing the proximal end of the housing while another side of the cannula (211) is configured with a luer end (212). Additionally, the base (210) can be further configured with luer lock (214) for a secured connection at the distal end of the connector (100). Luer lock (214) herein can be represented by an extended portion longitudinal to the tubular body (208), in which the portion has a screwthreaded internal wall surrounding the luer end (212) of the cannula (211).

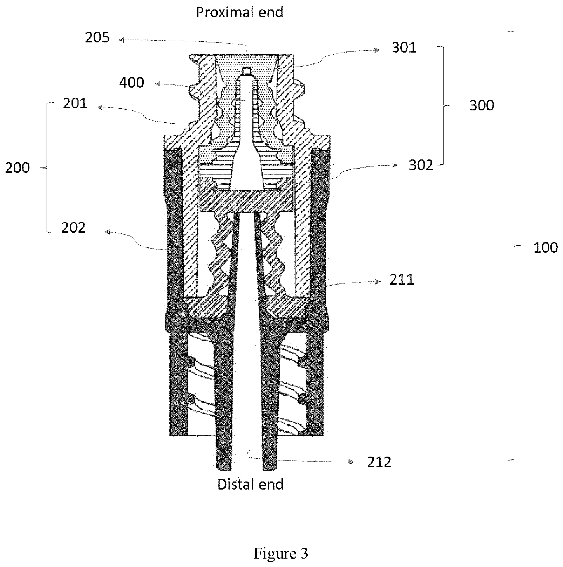

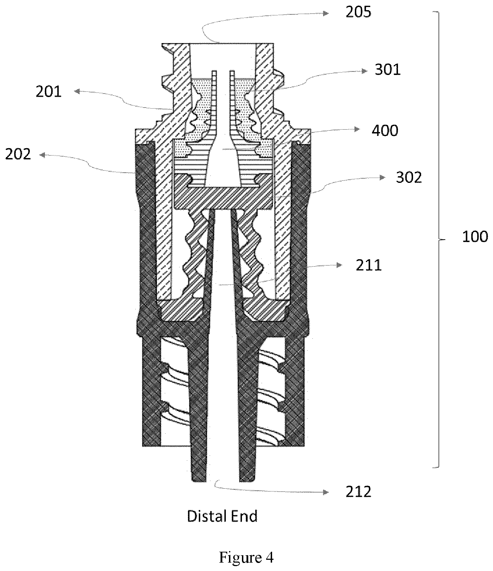

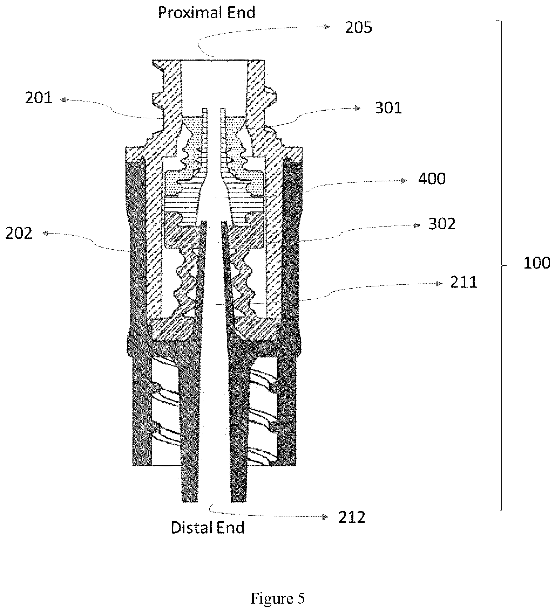

In one embodiment, the present invention includes a multi-stage fluid control valve provided within the first and second end pieces (201, 202). The multi-stage fluid control valve (300) is formed from an assembly of resiliently compressible components, referred hereinafter as first component (301) and second component (302). The resiliently compressible components are made of silicon rubber. Preferably, the resiliently compressible components includes a first component (301) which controls fluid flow primarily within the fluid passageway of the first end piece (201), and a second component (302) which primarily controls fluid flow through the second end piece (202). They are configured and arranged to work in association with one another, as well as other parts of the connector, to provide a fully relax (or close) condition as shown in Figure 3, a partial flow condition as shown in Figure 4, or fully open condition as shown in Figure 5 along the connector, when assembled.

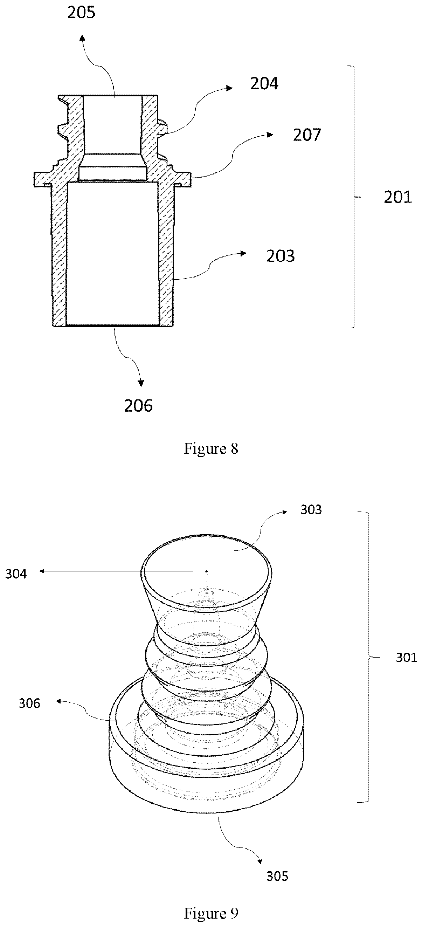

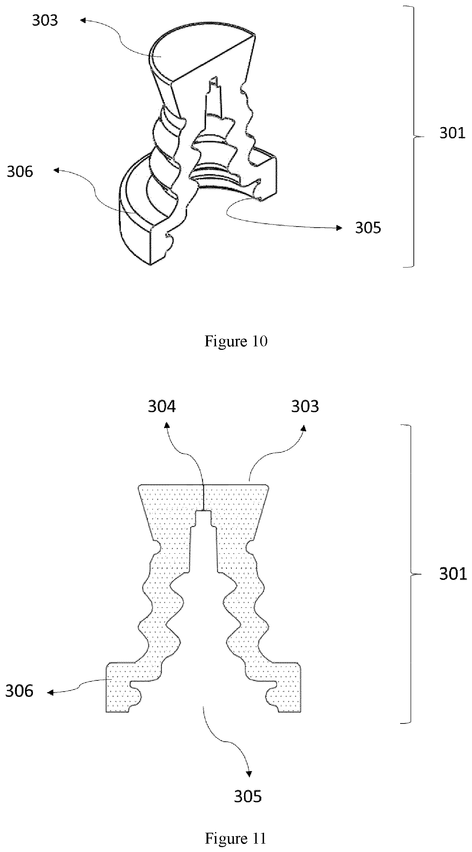

According to Figure 9 to 11, the first component (301) has a first hollow, corrugated body which facilitates compression and relaxation, whichever position the connector is desired to be used. One end of the corrugated body has a flat surface (303) with an orifice or slit (304) which can expand and open to allow fluid flow therethrough when compressed, and return to a closed position when relaxed. Another end of the corrugated body has an opening (305) exposing the hollow, internal side of the body. Preferably, the first component (301) has a size and external surface corresponding to, and slidable against, internal surface of the first end piece (201). More particularly, the first component (301) can be inserted into the first end piece (201), in vicinity to the extended neck portion (204). Upon inserted, the end with the flat surface also serves as the proximal end of the connector that is reachable and connectable to an external device. In one embodiment, the opening end (305) has an extended portion forming a first engaging member (306), in which the first engaging member (306) is larger in diameter than the end with the flat surface (303) thereby substantially engaging the first component (301) within the housing, especially within the first end piece (201) at the neck portion (204).

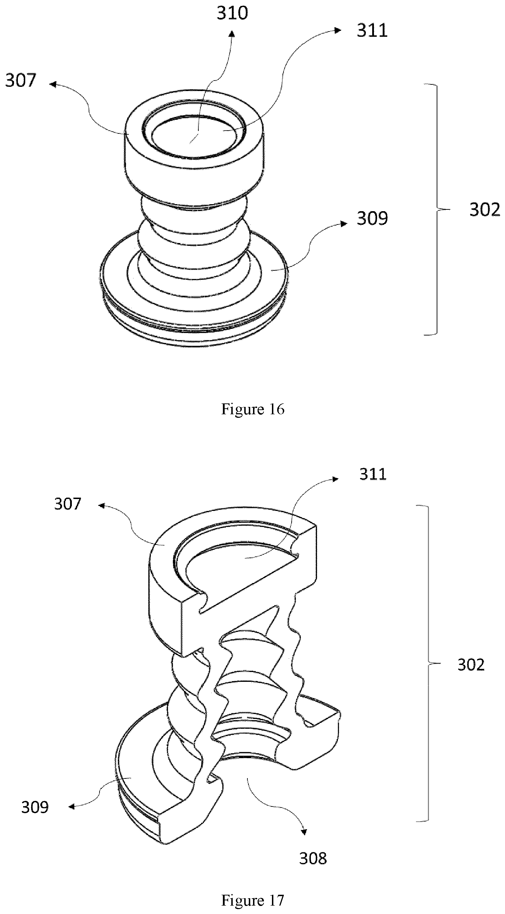

According to Figure 16 to 18, the second component (302) is a second hollow, corrugated body which also facilitates compression and relaxation, whichever position the connector is desired to be used. According to Figure 2 to 5, the second component (302) is arranged below the first component (301), in vicinity to the distal end of the connector. One end (307) of the body is closed while another end (308) is open and exposing the hollow, internal side of the body. Preferably the open end (308) of the body has a larger diameter than the closed end (307). Additionally, the open end (308) is configured with a second engaging member (309) which extends circumferentially, wherein upon assembled, the second engaging member (309) may serve as a seal between the first end piece (201) and the second end piece (202) at the base (210). In one embodiment, the closed end (307) is configured with an orifice or a slit (310) which can expand and open to allow fluid flow therethrough when compressed, and return to a closed position when relaxed. In one embodiment, the closed end (307) of the body is configured with a depression (311) to receive a coaxial conduit element (400) which will be described later. In one embodiment, the second component (302) is configured to shield the internal cannula (211) of the second end piece (202), upon assembly, as shown in Figure 2. Upon compressed, the orifice or slit (310) may open and allow fluid flow there through hence entering or leaving the internal cannula (211). In one embodiment, the second component (302) is configured to provide a closed space when it is in relax position within the housing, whereby backward flow from the distal end of the connector to the proximal end of the connector can be trapped therein.

In accordance to the preceding description, the fluid control valve, when inactive, closes fluid flow through the fluid passageways of both end pieces (201, 202) and hence through the connector (100), said control valve comprising a resiliently compressible assembly which can be activated such that a first predetermined compression of the resilient assembly puts the control valve into a partial flow condition (Figure 4) in which fluid flow is established into the fluid passageway of one a first of the end pieces (201), but holds fluid flow from egress through the fluid passageway of the opposite second end piece (202), and such that a continued further second predetermined compression of the resilient assembly puts the control valve into a fully open condition (Figure 5) allowing fluid flow through the fluid passageways of both the first (201) and second end pieces (202) and hence through the connector (100), and wherein disconnection of the connector (100) from said fluid pathway is affected by causing a multi-stage relaxation of the resilient assembly to a fully relaxed condition (Figure 3) to close fluid flow through the connector (100).

In one embodiment, there is a coaxial conduit element (400) housed within the first end piece (201) and disposed between the first (301) and second components (302) of the resilient assembly, and wherein fluid flow through one end of the conduit element is controlled by the first component (301) and fluid flow through the opposite end of the conduit element is controlled by the second component (302). A fluid connector having the abovementioned arrangement is such that the first predetermined pressure causes compression of the first component to open fluid communication of the first end piece (201) through the conduit element (400) and wherein the second predetermined pressure causes axial movement of the conduit element (400) and compression of the second component (302) to establish fluid flow through both end pieces (201, 202) and hence the connector (100). Additionally, the second predetermined pressure causes fluid communication between the opposite end of the conduit element (400) and fluid passageway of the second end piece (202) of the connector.

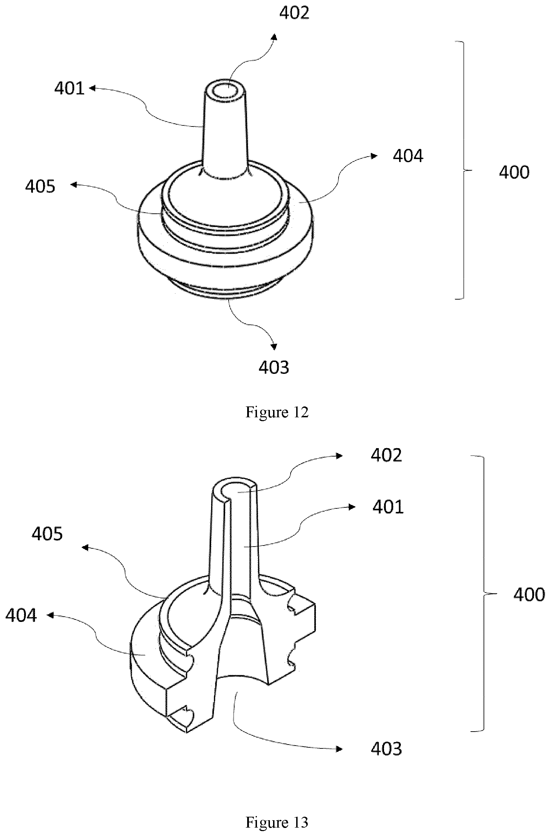

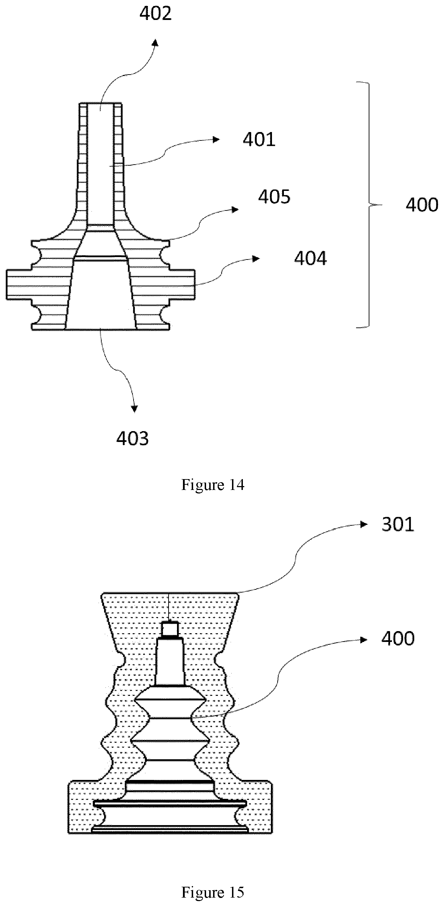

According to Figure 12 to 15, the coaxial conduit element (400) has an upright conduit (401) with a protruded end configured with a hole (402); and an opposite end having a relatively wider opening (403) than the pointed end (401). The conduit (401) extends from the hole (402) to the wider opening (404). Further, the conduit element (400) has an extended annular portion (404) and a raised annular portion (405) external to the conduit (401), both serve to secure an engagement when assembled. Upon assembly, the end with wider opening (404) is positioned and fitted into the depression (311) of the second component (302), such that that opening (404) substantially covers the orifice or slit (310) of the second component (302). On the other hand, the protruded end with hole (402) is positioned and fitted into the first component (301), in which the raised annular portion (405) is horizontally aligned with the engaging member (306) upon fitted, according to Figure 15. Upon assembled and receiving a first predetermined compression of the connector, the connector is in a partial flow condition (Figure 4) whereby the protruded end of conduit element (400) pushes the orifice or slit (304) of the first component (301) to an open condition while the wider opening (403) remains closed. Upon assembled and receiving a second predetermined compression of the connector, the connector achieves a fully open condition whereby the internal cannula (211) pushes the orifice or slit (310) from the second component (302) to an open condition, in which fluid within may flow in a straight pathway within the end pieces, through the connector (100).

Overall, a first predetermined compression of the resilient assembly puts the control valve into a partial flow condition (Figure 4) in which fluid flow is established into the fluid passageway of one a first of the end pieces (201), but holds fluid flow from egress through the fluid passageway of the opposite second end piece (202), and such that a continued further second predetermined compression of the resilient assembly puts the control valve into a fully open condition (Figure 5) allowing fluid flow through the fluid passageways of both the first (201) and second end pieces (202) and hence through the connector (100), and wherein disconnection of the connector (100) from said fluid pathway is affected by causing a multi-stage relaxation of the resilient assembly to a fully relaxed condition (Figure 3) to close fluid flow through the connector. More particularly, a release of the second predetermined compression puts a fully opened control valve into a partial flow condition. In this condition, the connector is able to prevent transfer or suction of bodily fluid from the distal end to the proximal end when, for example, there is a disconnection of external device from the proximal end. Then, a further release of the first predetermined compression puts the control valve into a fully relaxed condition.

The first and second compression herein are predetermined based on design of the multi-stage fluid control valve (300). More particularly, the material and/or dimension used for the components (301,302) can be adjusted during manufacture. It shall be understood that the material and/or dimension used provide sufficient rigidity and compressibility so that the multi-stage fluid control valve (300) will not have leakage upon use.

The present invention may be embodied in other specific forms without departing from its essential characteristics. The described embodiments are to be considered in all aspects only as illustrative and not restrictive. The scope of the invention is, therefore indicated by the appended claims rather than by the foregoing description. All changes, which come within the meaning and range of equivalency of the claims, are to be embraced within their scope.

## Claims

1. A fluid connector (100) particularly for medical use as an interconnector in a fluid pathway, the fluid connector, when assembled, including;
two coaxial, interconnected end pieces (201, 202) which comprise the opposite ends of the connector, and through each of which extends a fluid passageway within each end piece, said passageways together providing fluid communication between said opposite ends,
a multi-stage fluid control valve (300) provided within the interconnected end pieces (201, 202), the fluid control valve, when inactive, closes fluid flow through the fluid passageways of both end pieces (201, 202) and hence through the connector (100), said control valve comprising a resiliently compressible assembly (301, 302) which can be activated such that
a first predetermined compression of the resilient assembly (301, 302) puts the control valve (300) into a partial flow condition in which fluid flow is established into the fluid passageway of one a first of the end pieces (201), but holds fluid flow from egress through the fluid passageway of the opposite second end piece (202), and such that
a continued further second predetermined compression of the resilient assembly (301, 302) puts the control valve (300) into a fully open condition allowing fluid flow through the fluid passageways of both the first and second end pieces (201, 202) and hence through the connector (100), and wherein
disconnection of the connector (100) from said fluid pathway is affected by causing a multi-stage relaxation of the resilient assembly (301, 302) to a fully relaxed condition to close fluid flow through the connector (100).

2. A fluid connector according to claim 1 wherein the first end piece (201) is received at least partially within the second end piece (202), and wherein the first end piece (201) accommodates the resiliently compressible assembly (301, 302).

3. A fluid connector according to claim 1 or claim 2 wherein the resiliently compressible assembly includes a first component (301) which controls fluid flow primarily within the fluid passageway of the first end piece (201) and a second component (302) which primarily controls fluid flow through the second end piece (202).

4. A fluid connector according claim 4 wherein the first component (301) is resiliently movable within the first end piece (201) to open and close fluid flow therethrough and wherein the second component (302) is resiliently movable also within the first end piece (201) to open and close fluid flow through the second end piece (202).

5. A fluid connector according to claim 3 or claim 4 wherein a portion of the fluid passageway of the second end piece (202) extends within, and thereby provides a portion of, the fluid passageway of the first end piece (201).

6. A fluid connector according to claim 5 wherein the second component (202) opens and closes fluid communication through the passageway portion extending within the first end piece (201) which thereby controls fluid flow through both first and second end pieces (201, 202).

7. A fluid connector according to any of claims 4 to 6 wherein a coaxial conduit element (400) is housed within the first end piece (201) and disposed between the first (301) and second components (302) of the resilient assembly, and wherein fluid flow through one end of the conduit element (400) is controlled by the first component (301) and fluid flow through the opposite end of the conduit element (400) is controlled by the second component (302).

8. A fluid connector according to claim 7 wherein the arrangement is such that the first predetermined pressure causes compression of the first component (301) to open fluid communication of the first end piece (201) through the conduit element (400) and wherein the second predetermined pressure causes axial movement of the conduit element (400) and compression of the second component (302) to establish fluid flow through both end pieces (201, 202) and hence the connector.

9. A fluid connector according to claim 8 wherein the second predetermined pressure causes fluid communication between the opposite end of the conduit element (400) and fluid passageway of the second end piece (202) of the connector.
